(19) **Europäisches Patentamt / European Patent Office / Office européen des brevets**

(11) **EP 2 175 778 B1**

(12) **EUROPEAN PATENT SPECIFICATION**

(45) Date of publication and mention
of the grant of the patent:
**15.05.2019 Bulletin 2019/20**

(51) Int Cl.:
***A61B 5/103*** (2006.01)

(21) Application number: **08766842.2**

(22) Date of filing: **25.06.2008**

(86) International application number:
**PCT/NL2008/050419**

(87) International publication number:
**WO 2009/002170 (31.12.2008 Gazette 2009/01)**

(54) **METHOD FOR DETERMINATION OF A POTENTIAL MUTATION**

VERFAHREN ZUR BESTIMMUNG EINER MÖGLICHEN MUTATION

PROCÉDÉ PERMETTANT DE DÉTERMINER UNE MUTATION POTENTIELLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.06.2007 PCT/NL2007/050317**
**08.02.2008 EP 08151226**

(43) Date of publication of application:
**21.04.2010 Bulletin 2010/16**

(73) Proprietor: **Erasmus University Medical Center Rotterdam**
**3015 GE Rotterdam (NL)**

(72) Inventors:
• **CASPERS, Peter Jacobus**
**NL-3015 GE Rotterdam (NL)**
• **THIO, Hok Bing**
**NL-3015 GE Rotterdam (NL)**
• **PUPPELS, Gerwin Jan**
**NL-3015 GE Rotterdam (NL)**
• **KEMPERMAN, Patrick M.J.H.**
**NL-3015 GE Rotterdam (NL)**
• **NEUMANN, Hendrik Arent Martino**
**NL-3015 GE Rotterdam (NL)**

(74) Representative: **V.O.**
**P.O. Box 87930**
**2508 DH Den Haag (NL)**

(56) References cited:
**US-A1- 2003 109 774    US-A1- 2004 193 019**

• **KEMPERMAN P ET AL: "Loss-of-function mutations in the filaggrin gene predicted by in vivo Raman spectroscopy" JOURNAL OF INVESTIGATIVE DERMATOLOGY, [Online] vol. 128, 28 February 2008 (2008-02-28), pages 2117-2119, XP002495881 ISSN: 1523-1747 Retrieved from the Internet: URL:http://www.nature.com/jid/journal/v128 /n8/pdf/jid200829a.pdf> [retrieved on 2008-09-15]**
• **CASPERS P J ET AL: "In Vivo Confocal Raman Microspectroscopy of the Skin: Noninvasive Determination of Molecular Concentration Profiles" JOURNAL OF INVESTIGATIVE DERMATOLOGY, NEW YORK, NY, US, vol. 116, no. 3, March 2001 (2001-03), pages 434-442, XP002220321 ISSN: 0022-202X cited in the application**
• **RAWLINGS ANTHONY V ET AL: "Stratum corneum moisturization at the molecular level" JOURNAL OF INVESTIGATIVE DERMATOLOGY, vol. 103, no. 5, 1994, pages 731-740, XP002471925 ISSN: 0022-202X**
• **PALMER COLIN N A ET AL: "Common loss-of-function variants of the epidermal barrier protein filaggrin are a major predisposing factor for atopic dermatitis" NATURE GENETICS, NEW YORK, NY, US, vol. 38, no. 4, April 2006 (2006-04), pages 441-446, XP002444720 ISSN: 1061-4036**
• **CHRIT L ET AL: "In vivo chemical investigation of human skin using a confocal Raman fiber optic microprobe" J BIOMED OPT; JOURNAL OF BIOMEDICAL OPTICS JULY/AUGUST 2005, vol. 10, no. 4, July 2005 (2005-07), XP002471926**

## Description

FIELD OF THE INVENTION

The invention is directed to a method for non-invasive determination of the potential presence of one or more loss-of-function mutation(s) in the gene encoding for filaggrin.

BACKGROUND OF THE INVENTION

Atopic dermatitis (AD) is a major problem in dermatology. Estimates for the prevalence of atopic dermatitis in developed countries range between 15 % and 20 %. [Roll et al. Curr. Opin. Allergy Clin. Immunol. 2004, 4(5), 373-378] Atopic dermatitis represents an enormous burden on health care in general.

The skin is divided in two layers, the dermis and the epidermis. The outermost layer of the epidermis, the stratum corneum, is the main protective barrier of the body against water loss and penetration of harmful agents. An impaired barrier function is likely to be a primary event in atopic dermatitis.

Atopic dermatitis is a common chronic inflammatory skin disease characterized by itchy, inflamed skin. [Roll et al. Curr. Opin. Allergy Clin. Immunol. 2004, 4(5), 373-378 and Stemmler et al. J. Invest. Dermatol. 2007, 127, 722-724] It is also well known that a predisposition for atopic dermatitis impairs the ability of a person to be active in certain professions such as hairdressing and cookery.

Current opinion is that early detection of the predisposition of young children to develop atopic dermatitis and targeted treatment of the skin barrier can prevent further development of the atopic syndrome.

The protein filaggrin is of crucial importance for the formation and maintenance of the skin barrier. The protein is necessary in giving the correct macrostructure to the keratin fibrils and it provides the amino acids for the production of the natural moisturising factor (NMF).

Loss-of-function mutations in the gene encoding for filaggrin have been implicated in atopic dermatitis. [Roll et al. Curr, Opin. Allergy Clin. Immunol. 2004, 4(5), 373-378; Stemmler et al. J. Invest. Dermatol. 2007, 127, 722-724; Sandilands et al. J. Invest. Dermatol. 2006, 126, 1770-1775; Sandilands et al. Nature Genetics 2006, 38, 337-342; and Smith et al. Nature Genetics 2007, 39, 650-654] Partial or complete loss of the ability to produce filaggrin results in an impaired barrier function. As a consequence, the body becomes more susceptible to allergens exposure, which may result in atopic dermatitis. Loss-of-function mutations in the filaggrin gene are strong predisposing factors for atopic dermatitis and asthma, which is a major and increasing problem in the developed nations. [Sandilands et al. J. Invest. Dermatol. 2006, 126, 1770-1775; Sandilands et al. Nature Genetics 2006, 38, 337-342; Smith et al. Nature Genetics 2007, 39, 650-654; and Irvine et al. J. Invest. Dermatol. 2006, 126, 1200-1202] However, although there is a correlation between loss-of-function mutation in the filaggrin gene and atopic dermatitis, there is no direct link. People without the gene mutation may develop atopic dermatitis. People with the gene mutation do not necessarily have atopic dermatitis.

Approximately 10 % of the people of European origin carry a mutation in the filaggrin gene. [Sandilands et al. J. Invest. Dermatol. 2006, 126, 1770-1775] Homozygous filaggrin gene mutants exhibit complete loss of the ability to produce filaggrin.

Known methods to determine mutations in the filaggrin gene use specialised genotyping techniques, see for instance US-A-2003/0 124 553. Specific biochemical assays are required to detect the specific mutations in the gene encoding for filaggrin. Genotyping techniques require multiple cycles of PCR (polymerase chain reaction) amplification and sequencing to isolate and amplify the filaggrin-encoding DNA. Each filaggrin mutation is then analysed by a specific genotyping assay. Most of these assays are not commercially available and several laboratories working on the filaggrin gene mutations have developed their own assays. Analysis of 15 variants of the filaggrin mutation is described by Sandilands et al. in Nature Genetics 2006, 38, 337-342. These genotyping methods are expensive and time-consuming and require highly specialised laboratory facilities and personnel. Genotyping methods are unsuitable for population screening purposes.

Accordingly, a need exists for a quick and easy-to-use method to screen the population for the potential presence of loss-of-function mutations in the filaggrin gene.

Further, there is a need for a quick and easy-to-use method to determine whether an atopic dermatitis patient has a loss-of-function mutation in the filaggrin gene for a correct diagnosis of the disease and choice of appropriate treatment.

By means of *in vivo* Raman spectroscopic measurements of the stratum corneum it is possible to determine the relative concentration of a complex of amino acids and amino acid derivatives, collectively referred to as the natural moisturizing factor (NMF), the source of which is primarily the protein filaggrin. Filaggrin is present in a narrow region in the lower stratum corneum, where it is enzymatically degraded to contribute to the NMF. [Scott et al. Biochim. Biophys. Acta 1982, 719(1), 110-117 and Rawlings et al. J. Invest. Dermatol. 1994, 103, 731-741] Loss-of-function mutations in the gene encoding for filaggrin thus contribute to the NMF content in the skin. However, the NMF content in the skin is determined by many other factors, some having a much larger impact than a loss-of-function gene mutation.

Thus, although it may be true that a loss-of-function mutation in the filaggrin-gene leads to a lower NMF content in the skin, the inverse is certainly not a given. An abnormally low NMF content is not necessarily caused by a gene mutation, because the process of gene transcription and production of pro-filaggrin and its enzymatic processing into filaggrin and the subsequent enzymatic/proteolytic degradation of filaggrin to NMF are affected by many factors. This, among other matters, causes the NMF content to strongly differ from one body location to the next, despite the fact that the same functional pro-filaggrin gene is present everywhere. The NMF content is furthermore affected by factors such as age, bathing, washing with soap, and environment. This is for instance illustrated by Rawlings et al. [J. Invest. Dermatol. 1994, 103, 731-741], who describe the process of NMF-creation through enzymatic and proteolytic degradation of filaggrin (which in itself is the product of enzymatic processing of profilaggrin, the protein encoded by the filaggrin-gene). Rawlings *et al.* describe a correlation between disease states and absence of NMF and that the inability to produce NMF might be a critical mechanism contributing to skin problems ranging from simple xerosis to severe psoriasis. Furthermore, Rawlings *et al.* mention that washing washes out most of NMF from superficial stratum corneum. In addition, they note a significant age-related decline, by more than a factor of two, in stratum corneum NMF content, which is attributed to lower synthetic activity. Also, they state that the conversion of filaggrin to NMF is dependent on water activity and that the stratum corneum adjusts this process to the environmental conditions to which it is exposed. The NMF content has been found to vary strongly between individuals with clinically normal skin. Variations as a function of age and seasonal influences have also been found, as have variations in relation to location on the body. [Van der Pol et al. Abstract IFSCC meeting, Amsterdam, The Netherlands, 24-27 September 2007 and Van der Pol et al. Abstract ISBS National meeting, Stone Mountain GA, USA, 12-14 October 2006].

It is furthermore unclear how effects such as abnormal desquamation, which have been described for atopic dermatitis, affect the NMF content in the skin.

Clinical signs of efficacy of a treatment of a skin condition sometimes become observable only long after the start of a treatment. For example, under Dutch guidelines for treatment of psoriasis with Raptiva ® (efalizumab, Merck Serono International S.A., Geneva, Switzerland), treatment is continued for 12 weeks before the clinician decides to continue or discontinue the treatment. However, clinical improvement lags behind the underlying molecular changes. A method to monitor molecular changes such as changes in the NMF content would therefore offer the possibility to determine the efficacy of a skin treatment much faster than based on conventional clinical assessment.

It is an object of the invention to provide for a method for rapid non-invasive determination of the likelihood of the presence of one or more loss-of-function mutations in the filaggrin gene in a subject, particularly a human subject.

It is a further object of the invention to provide for a rapid and objective method to distinguish between a homozygous and a heterozygous loss-of-function mutation in the filaggrin gene.

It is a further object of the invention to provide for a rapid and objective method to constitute suitable panels of individuals for studies concerning determination of penetration and/or effects of topically applied products, *i.e.* to provide objective measures to include or exclude individuals from study groups.

It is a further object of the invention to provide for an objective method to determine whether an individual is unsuitable, or at least less suitable, for a certain profession or activity because of an increased risk to develop an occupational skin problem.

It is a further object of the invention to provide for an objective method facilitating population screening to determine whether a young child has a predisposition for skin conditions related to one or more loss-of-function mutation(s) in the filaggrin gene, and thereby enable early intervention with preventive treatment and/or direct the patient to further diagnostic tests.

It is a further object of the disclosure to provide for an objective method to determine whether an individual suffering from atopic dermatitis has a skin barrier impairment due to a filaggrin defect and to adjust the therapy accordingly.

It is a further object of the disclosure to provide for an objective method to determine the efficacy of a therapeutic treatment of atopic dermatitis.

It is a further object of the disclosure to provide for an objective method to determine the efficacy of a therapeutic treatment of atopic dermatitis before clinical signs of improvement can be observed.

It is a further object of the disclosure to provide for a rapid and objective method to predict the outcome of a therapy or treatment for skin disease.

It is a further object of the disclosure to provide for a rapid and objective method to predict the effects of skin treatment with a personal care product.

It is a further object of the disclosure to provide for a rapid and objective method to guide choice of appropriate treatment or therapy of a skin disease.

It is a further object of the disclosure to provide for a rapid and objective method to guide selection of a personal care product for skin care.

It is a further object of the disclosure to provide for a rapid and objective method to predict the effects of skin exposure to certain environmental conditions, chemicals, and other matter.

SUMMARY OF THE INVENTION

The invention is directed to a method for non-invasive determination of the potential presence of one or more loss of function mutation(s) in the gene encoding for filaggrin of an individual according to claim 1

In addition, the invention is directed to a method as further specified in the claims

BRIEF DESCRIPTION OF THE DRAWINGS

Figure 1 is a Raman spectrum of the stratum corneum of the arm of an individual without a filaggrin mutation. The black areas (numbered 1 and 2) indicate Raman bands from NMF. The grey area (number 3) indicates a Raman band from stratum corneum tissue.

Figure 2 is a Raman spectrum of the stratum corneum of the arm of an individual with a filaggrin mutation. The black areas (numbered 1 and 2) indicate Raman bands from NMF. The grey area (number 3) indicates a Raman band from stratum corneum tissue.

Figure 3 is a Raman spectrum of the stratum corneum, which illustrates one method to determine the NMF content from the vibrational signal.

Figure 4 is a Raman spectrum of natural moisturising factor.

Figure 5 is a Raman spectrum of human skin.

Figure 6 is a skin depth profile of natural moisturising factor of an individual without a loss of function mutation of the filaggrin gene

Figure 7 is a skin depth profile of natural moisturising factor of an individual with a loss of function mutation of the filaggrin gene.

Figure 8 shows the relative natural moisturising factor content in the arm of individuals without a loss of function mutation of the filaggrin gene. The dashed line indicates the median NMF content.

Figure 9 shows the relative natural moisturising factor content in the arm of individuals with a loss of function mutation of the filaggrin gene. The dashed line indicates the median NMF content in the arm of individuals without a loss of function mutation of the filaggrin gene.

Figure 10 shows the relative natural moisturising factor content in the thenar of individuals without a loss of function mutation of the filaggrin gene. The dashed line indicates the median NMF content.

Figure 11 shows the relative natural moisturising factor content in the thenar of individuals with a loss of function mutation of the filaggrin gene. The dashed line indicates the median NMF content in the thenar of individuals without a loss of function mutation of the filaggrin gene.

DETAILED DESCRIPTION OF THE INVENTION

The term "vibrational spectroscopy" as used herein is defined as any spectroscopic technique that allows the analysis of vibrational and/or rotational modes of a molecule.

The term "Raman spectroscopy" as used herein is defined as a spectroscopic technique used to study vibrational and/or rotational modes in a system, and relies on inelastic scattering (also referred to as Raman scattering) of monochromatic light, usually from a laser in the visible, near infrared, or near ultraviolet range. The incident laser light can lose or gain quanta of vibrational and/or rotational energy from the system, which results in a change of energy of the laser photons. This change in energy of the laser photons causes a spectral shift and provides information on the vibrational and/or rotational modes in the system. Typically, a sample is illuminated with a laser beam. Light from the illuminated spot is collected with a lens and sent through a spectrometer. Wavelengths close to the laser line (due to elastic Rayleigh scattering) are filtered out and those in a certain spectral window away from the laser line are dispersed onto a detector.

A Raman spectrum is a set of very narrow spectral lines emitted from object molecules when illuminated by an incident light. The width of each spectral line is strongly affected by the spectral width of the incident light and hence tightly monochromatic light sources, such as lasers, are used. The wavelength of each Raman line is expressed as a wavenumber-shift from the incident light, which is the difference between the wavenumber of the Raman line and the incident light. The wavenumber-shift, not the absolute wavenumber, of the Raman lines is specific to particular atomic groups in molecules. Raman spectra measure the vibrational and/or rotational modes of molecules which are determined by their molecular structure, especially by atomic groups such as methylene, ethylene, amide, phosphate or sulphide.

Most applications of Raman spectroscopy in biology are concerned with change in vibrational and/or rotational modes of macromolecules or related small molecules. Changes in either the wavenumber-shift of single Raman lines or the relative intensities of two or more Raman lines in an atomic group have been interpreted as indicating conformational changes in macromolecules. For these reasons Raman spectroscopy is mainly used for qualitative studies of molecules and molecular dynamics in biology. For easier and clearer interpretation of Raman spectra, use of the technique has been restricted mainly to purified materials and their systems, such as enzyme reactions. However, because Raman spectra are based on the specific vibrations and/or rotations of atomic groups they can also be used to characterise and quantify a mixture of molecules as compositions of atomic groups by a method akin to fingerprinting. Although unable to completely resolve the composition of a sample in terms of a list of chemical compounds, but for the most abundant molecular species, it does give a rough sketch of the molecular composition of the natural environment and how it changes with time.

The term "local natural moisturising factor content" as used herein is defined as the NMF content or NMF concentration in the stratum corneum at a given location on the body, such as the volar aspect of the forearm or the thenar (palm of the hand) and at a given distance below the skin surface.

The invention is based on the insight that, although many factors influence the NMF concentration of NMF content in the corneum stratum of the skin and thereby mask the effect of a loss-of-function gene mutation on the NMF content, there are locations on the body of an individual where the NMF concentration is relatively unaffected by influences and processes other than loss-of-function profillagrin gene mutation. With this insight, a suitable choice of measuring technique allows to obtain a reliable diagnosis that an individual has a loss-of-function mutation in the filaggrin gene, and accordingly an increased risk to atopic dermatitis, asthma, allergic rhinitis *etc.* It was found that the defect in filaggrin production leads to a lower than normal concentration of NMF in the stratum corneum of an individual, which can be detected by vibrational spectroscopy. It has not been previously suggested to use the NMF concentration as a method for determining the presence of a gene mutation, and it is surprising that this appears possible.

Locations of the body of an individual at which the one or more loss-of-function mutation(s) in the filaggrin gene have a stronger influence on the NMF concentration than other factors which influence the NMF concentration can be found as follows.

After selecting a trial body location, a vibrational spectrum from the stratum corneum at this trial body location of a group of one or more individuals with a known loss-of-function filaggrin gene mutation is obtained and the natural moisturising factor content at the trial body location is determined from analysis of the vibrational spectrum. A vibrational spectrum is also obtained from the stratum corneum at this trial body location of a group of one or more individuals without a loss-of-function filaggrin gene mutation. Again, the natural moisturising factor content at the trial body location is determined from analysis of the vibrational spectrum. Finally, it is determined whether at the trial body location loss-of-function mutations in the gene encoding for filaggrin have a stronger influence on the natural moisturising factor concentration than other factors influencing the natural moisturising factor concentration by assessing whether the natural moisturising factor content in the stratum corneum at the trial body location in the group of individuals with a known loss-of-function fillagrin gene mutation is lower than in the group of individuals without a loss-of-function fillagrin gene mutation. The group of individuals with a known loss-of-function fillagrin gene mutation and the group of individuals without a loss-of function fillagrin gene mutation preferably each comprise at least six individuals, more preferably at least twelve individuals.

The method of the invention allows the distinction between a heterozygous carrier of a filaggrin mutation, which can result in a moderately decreased detectable NMF content in the stratum corneum, and a homozygous carrier of the filaggrin mutation, which may result in a substantially complete absence of detectable NMF in the stratum corneum.

According to the method of the invention a vibrational spectrum, or selected parts thereof, of the stratum corneum of the individual is measured. The vibrational spectrum may be measured using any vibrational spectroscopy technique. The preferred spectroscopic method is Raman spectroscopy. Other spectroscopy methods include infrared spectroscopy, such as near-infrared spectroscopy, Fourier transform infrared (FT-IR) spectroscopy, or attenuated total reflection (ATR) infrared spectroscopy, for example as described by Bommannan et al. J. Invest. Dermatol. 1990, 95(4), 403-408.

The vibrational spectrum is a representation of the molecular composition of a sample. The NMF content in the stratum corneum can be determined from the vibrational spectrum in any of the following ways.

In one embodiment, the NMF content is determined as the intensity of the vibrational signal of NMF relative to the intensity of the vibrational signal of an internal reference. A suitable internal reference is for instance the signal of keratin. Accordingly, the NMF content may be determined as the ratio of the NMF-signal intensity to the keratin-signal intensity. However, also other common constituents of the skin, not belonging to the group of molecular compounds that together constitute the NMF, can be used as suitable internal reference. For example such an internal reference may be the vibrational signal of a sample of stratum corneum tissue after removal of all or most of the molecular compounds that together constitute the NMF. A method to extract NMF from a sample of stratum corneum has been described in Caspers et al. J. Invest. Dermatol. 2001, 116, 434-442. In this article the possibility to detect interpersonal variations in NMF content is suggested, as well as the use of Raman spectroscopy to study differences in molecule concentrations in the skin as a result of disease processes and treatments, without further specification.

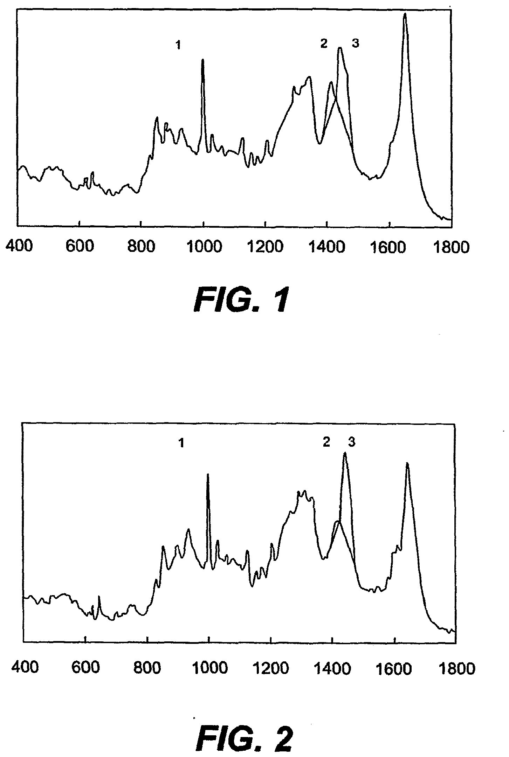

Signal intensities can suitably be determined from the vibrational signal in one or more wavelength intervals,

in which NMF signal contributions are dominant and one or more wavelengths intervals in which the internal reference signal contributions are dominant. The NMF signal intensity may then be determined from an area $A_1$ under the curve in one or more wavelength intervals in which the signal contribution of NMF is dominant (see Figures 1 and 2, areas numbered 1 and 2). The reference signal intensity may be determined from an area $A_2$ under the curve in of one or more wavelength intervals in which the signal contribution from the internal reference is dominant (see Figures 1 and 2, area numbered 3). Accordingly, a useful measure of the NMF content (R) may be obtained from the ratio: $R = A_1/A_2$.

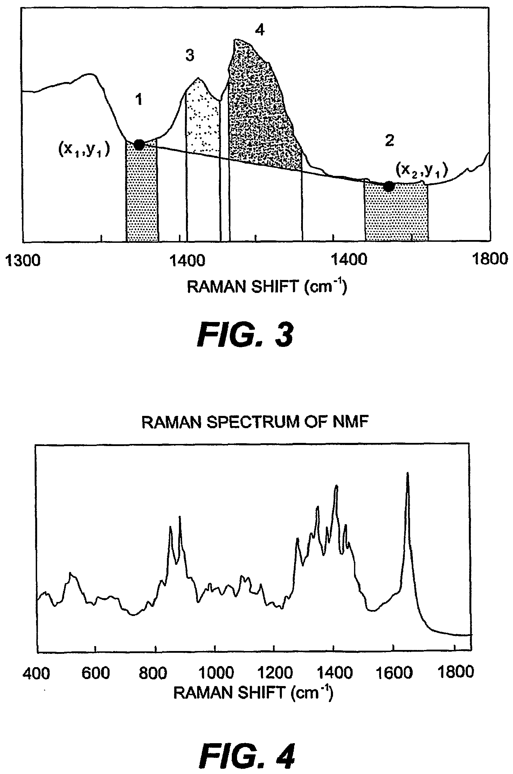

In another embodiment, signal intensities are measured in one or more wavelength intervals in which NMF signal contributions are dominant, in one or more wavelengths intervals in which the internal reference signal contributions are dominant, and in one ore more spectral intervals in which little or no Raman signal is anticipated and which may therefore serve to estimate background signal intensities. As illustrated in Figure 3, the NMF content may then be determined from an area $A_3$ under the curve of a dominant NMF signal and an area $A_4$ under the curve of the internal reference signal. The areas under the curve may be determined from the measured signal intensities in the different wavelength intervals according to the following expressions, wherein $I_n$ is the measured signal intensity in spectral interval n, $A_n$ is the area under the curve in spectral interval n, $x_n$ is the central wavenumber of spectral interval n, $y_n$ is the average signal intensity in spectral interval n, and $d_n$ is the width of spectral interval n (see Figure 3). Area $A_3$ under the curve for NMF can be determined using

$$A_3 = I_3 - d_3\left( y_1 + \frac{(y_2 - y_1)(x_3 - x_1)}{(x_2 - x_1)} \right) \tag{1}$$

Area $A_4$ under the curve for the internal reference signal can be determined using

$$A_4 = I_4 - d_4\left( y_1 + \frac{(y_2 - y_1)(x_4 - x_1)}{(x_2 - x_1)} \right) \tag{2}$$

A measure of the NMF content can then be obtained from the ratio between the areas under the curve $A_3$ and $A_4$:

$$C_{NMF} = A_3 / A_4$$

The person skilled will understand that other signal intensity ratios are possible to obtain an estimate of NMF content. For the method of this invention it is not important that the measure of NMF content determination scales linearly with NMF content. For instance a measure which scales monotonically with NMF content may suffice.

In a preferred embodiment, the NMF content is determined from the intensity of the vibrational signal of NMF, relative to the intensity of the vibrational signal of an internal reference. A suitable internal reference is for instance keratin.

In a preferred embodiment, the NMF content is determined by spectral fitting. From a reference set of vibrational spectra that comprise the vibrational spectrum of skin or most of the vibrational spectrum of skin, the contribution of each reference spectrum to the vibrational spectrum of skin is determined. The relative contributions may be determined by fitting the reference spectra or selected spectral regions of the reference spectra to the vibrational spectrum of skin or selected spectral regions of the vibrational spectrum of skin. The fit coefficients represent the relative contributions of each of the reference spectra. [Caspers et al. J. Invest. Dermatol. 2001, 116, 434-442] Preferably, one of the reference spectra is a spectrum of NMF. Alternatively, one or more reference spectra are spectra of constituents of NMF. The set of reference spectra may be collected *in vitro* from pure skin constituents, solutions of pure skin constituents and/or assemblies of pure skin constituents. A particularly preferred skin constituent that may be used for a reference spectrum is keratin.

In another embodiment, the NMF content is determined by calculation of the intensity of one or more peaks in the vibrational spectrum of NMF or in the spectra of constituents of NMF, and calculation of the intensity of one or more peaks in the spectrum of an internal reference. A suitable internal reference is for instance keratin. However, also other common constituents of the stratum corneum, not belonging to the group of molecular compounds that together constitute the NMF can be used as suitable internal reference.

Vibrational spectra, and more in particular Raman spectra, can be analysed automatically and in real-time on a personal computer, which is programmed to calculate the NMF content in the stratum corneum from a vibrational spectrum. This makes the result of the analysis instantly available. In another embodiment the vibrational spectra are stored and analysed at a later time.

Preferably, the NMF content is determined by recording vibrational spectra *in vivo*, directly on the skin. However, the NMF content can also be determined by recording vibrational spectra *ex vivo* on a stratum corneum sample that has been taken from the individual.

In the invention, the local NMF content is determined by measuring vibrational spectra as a function of the distance to the surface of the skin. This results in a so-called NMF depth profile.

In another embodiment the NMF content in the skin is determined by measuring vibrational spectra at a fixed and optimal distance from the skin surface at a given body location, preferably in the central part of the stratum corneum. The vibrational spectra of the stratum corneum on the thenar can be measured at a point 1-70 $\mu$m beneath the skin surface, preferably at a point 2-50 $\mu$m beneath the skin surface, more preferably at a point 3-30 $\mu$m beneath the skin surface. The vibrational spectra of the stratum corneum, on for example the volar aspect of the forearm, can be measured 1-10 $\mu$m beneath the skin surface, preferably 2-8 $\mu$m, more preferably 3-6 $\mu$m beneath the skin surface.

In another embodiment the overall NMF content in the stratum corneum is determined as an average NMF content across the full thickness of the stratum corneum or across a specific depth range. The NMF content can be measured at a number of different distances below the skin surface, for instance at three different distances below the skin. Preferably, when measuring on the thenar, the NMF content is measured at about 30, 40 and 50 $\mu$m beneath the skin surface. Preferably, when measuring on the inner forearm, the NMF content is measured at about 4, 6 and 8 $\mu$m beneath the skin surface. The NMF content measured at different depths can then be averaged to yield a single NMF content value.

In another embodiment the overall NMF content in the stratum corneum is measured as an average NMF content across the full or partial thickness of the stratum corneum. The Raman instrument can have a depth resolution which is equal in size, in the axial dimension, as the full or partial thickness of the stratum corneum. A vibrational spectra can be measured at a fixed and optimal distance from the skin surface at a given body location, preferably in the central part of the stratum. As a result a vibrational spectrum of the full or partial thickness of the stratum corneum can be measured in a single measurement.

In another embodiment the method is used to monitor changes, or the absence of changes, in the NMF content in the stratum corneum of a patient who is being treated for a skin condition which correlates with an abnormal NMF content, for example atopic dermatitis. In such an embodiment the method provides direct clinical information about the efficacy of the treatment.

One or more vibrational spectra can be measured in the stratum corneum of an individual. It is preferred that more than one spectrum is measured, for instance at least 2 spectra, preferably at least 5 spectra, and more preferably at least 10 spectra. Measurements of the NMF content may be repeated several times (such as 2-10 times) on slightly different locations, in order to average out the lateral biological variation in the NMF content. The slightly different locations can for instance be a translation over 0.05-1 mm, preferably 0.1-0.75 mm, more preferably 0.2-0.5 mm.

In a specially preferred embodiment, the vibrational spectra are recorded on the thenar of the individual. Another preferred location to perform the vibrational spectra is the volar aspect of the forearm of the individual. Yet, in principle the vibrational spectra may be recorded on any other part of the body surface of the individual.

Preferably, the vibrational spectra are measured by Raman spectroscopy. In a special embodiment an in vivo confocal Raman microspectrometer as described by Caspers *et al.* may be used to record the vibrational spectra. [Caspers et al. Biospectroscopy 1998, 4, 31-39 and Caspers et al. J. Invest. Dermatol. 2001, 116, 434-442] Another example of an in vivo confocal Raman microspectrometer is the model 3510 Skin Composition Analyzer (River Diagnostics, Rotterdam, The Netherlands). However, also a simple Raman spectrometer is suitable for carrying out the method of the invention.

In a simple and cheap embodiment the laser light is focused at a fixed distance from the skin surface. In the case of the relatively thick stratum corneum of the thenar in the order of 100 $\mu$m thick, this fixed distance can be at 1-70 $\mu$m below the skin surface, preferably 2-50 $\mu$m, more preferably 3-30 $\mu$m. In the case of thinner stratum corneum of 12-25 $\mu$m, such as the volar aspect of the forearm, the fixed distance can be at 1-10 $\mu$m, preferably 2-8 $\mu$m, more preferably 3-6 $\mu$m. This eliminates the need for an accurate dynamic focussing device, which is part of commercially available confocal systems, such as the Model 3510 Skin Composition Analyzer (River Diagnostics). Such an accurate dynamic focussing device would needlessly drive up the cost of instruments to perform the methods according to the present invention.

It is not required that the Raman spectrometer has a high spatial resolution. In fact, a moderate or low spatial resolution can be of advantage, since it enables signal collection from *e.g.* a single relatively large part of the stratum corneum in one measurement. Thus, simpler and less expensive optical components in the light delivery and the light detection path can be used.

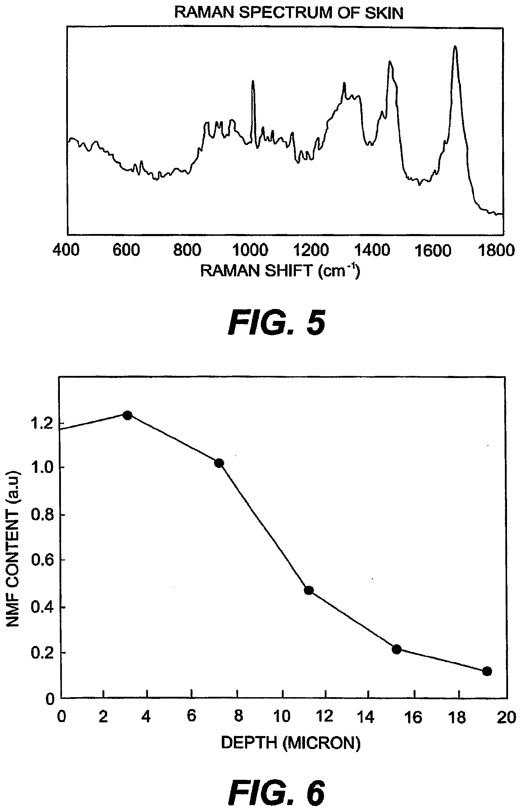

Further, a Raman spectrometer can be used, which detects several selected parts of the Raman spectrum with a low spectral resolution, and still provide sufficient information to distinguish between normal and aberrant NMF content. The main signal contributions from NMF occur in the three spectral windows 800-900 cm$^{-1}$, 1280-1480 cm$^{-1}$ and 1640-1660 cm$^{-1}$, see Figure 4. In a preferred embodiment the Raman signal of NMF is recorded in one or more of these spectral regions, and the Raman spectrum of the internal standard, see Figure 5, in a non- or partially overlapping spectral region.

A commercially available Raman skin analyser may be used. For example a Model 3510 Skin Composition Analyzer (River Diagnostics) may be used. This system was designed for rapid, non-invasive analysis of the molecular composition of the skin. The device enables measurement of Raman spectra of the skin at a range of depths below the skin surface, and thereby enables quantitative and semi-quantitative analysis of molecular concentrations or contents in the skin as a function of distance to the skin surface.

The method of the invention enables the determination whether a specific individual is unsuitable, or at least less suitable, for a certain profession (such as hair-dresser or cook) or activity.

The method of the invention further allows differentiation between a homozygous and a heterozygous loss-of-function mutation in the filaggrin gene of an individual by classifying the loss-of-function mutation as a function of the natural moisturising factor content. A moderately decreased detectable NMF content in the stratum corneum is an indication of a heterozygous carrier of a filaggrin mutation, while substantially complete absence of detectable NMF in the stratum corneum is an indication of a homozygous carrier of the filaggrin mutation.

The method can be used to predict whether a certain therapy or treatment of a patient, who is treated for a skin disease, which can be AD, or psoriasis, or another skin disease, is likely to have the desired effect. In this embodiment, the outcome of the treatment is correlated with the relative amount of NMF in the stratum corneum and the relative amount of NMF in the stratum corneum is used as a predictive marker for the effect of the treatment or therapy.

In this method, in a first step the method is used to determine the NMF content in the stratum corneum of a number of patients who receive treatment and a correlation is determined between the NMF content in the stratum corneum of the patients who receive the treatment and the effect of the treatment.

In a second step the NMF content in the stratum corneum of a patient is determined and used with the correlation that has been established in the first step, to predict the chance that the treatment of the patient will be successful.

In an optional third step the evaluation of possible treatments or therapies according to the first and second steps is used as a means for guiding the choice for a certain therapy or treatment by way of comparing the predicted outcomes of a number of potentially applicable treatments or therapies.

The method can also be used to predict the effect of the use of a certain personal (skin) care product. In this method, the outcome of the use of the personal (skin) care product is correlated with the relative amount of NMF in the stratum corneum, and the relative amount of NMF in the stratum corneum is used as a predictive marker for the effect of the use of the personal (skin) care product.

In a first step the method is used to determine the NMF content in the stratum corneum of a number of subjects who use the personal (skin) care product, and a correlation is determined between the NMF content in the stratum corneum prior to use of the product and the effect using said personal (skin) care product.

In a second step the NMF content in the stratum corneum of a subject is determined and used with the correlation that has been established in the first step, to predict the effect of the use of the personal (skin) care product.

In an optional third step the evaluation of possible treatments or therapies according to the first and second steps is used as a means to guide the choice for a certain personal care product by way of comparing the predicted outcomes of a number of personal care products.

The method can also be used to predict the effect of exposure of a person to certain environmental conditions, chemicals, or other matter. In this method, the outcome of such exposure is correlated with the relative amount of NMF in the stratum corneum, and the relative amount of NMF in the stratum corneum is used as a predictive marker for the effect of exposure.

In a first step the method is used to determine the NMF content in the stratum corneum of a number of subjects who are exposed to a certain environmental condition or conditions, chemicals, or other matter, and a correlation is determined between the NMF content in the stratum corneum prior to the exposure and the effect of the exposure.

In a second step the NMF content in the stratum corneum of a subject is determined and used with the correlation that has been established in the first step, to predict the effect of exposure to a certain environmental condition or conditions, chemicals, or other matter.

In an optional third step the evaluation of possible exposure according to the first and second steps is used as a means to provide advice with regard to exposures, *e.g.* with regard to avoiding certain types of exposure.

In a further aspect, the disclosure is directed to a method for treating an individual suffering from atopic dermatitis, comprising determining whether the individual has a potential presence of one or more loss-of-function mutation(s) in the gene encoding for filaggrin as described above, and adjusting the therapy based on the outcome of said determination. The therapy can for instance be adjusted by directing it more specifically to skin barrier impairment and can include administering of oral antihistamines, topical emollients, topical doxepin, topical corticosteroids, topical hydrocortisones, topical immunomodulators, and/or ultraviolet light therapy.

The method of the invention is an attractive and relatively cheap screening method. Based on the results from this screening it can be determined whether further (more expensive) screening or diagnosis is necessary.

**Example**

Measurements were carried out on a panel of individuals, consisting of 7 individuals without known loss-of-function mutations in the filaggrin gene and 6 individuals with a known loss-of-function mutation, either R501X or 2282del4, in the fillagrin gene.

For Raman measurements, an *in vivo* confocal Raman microspectrometer Model 3510 Skin Composition Analyzer was used. The individual placed the skin region of interest on a fused silica window mounted in the measurement stage. Laser light was focused in the skin with a microscope objective located under the window. The distance of the laser focus to the skin surface was controlled by the instrument control software (RiverICon, River Diagnostics). After the start and end points and the incremental step size had been defined, the software automatically recorded a depth profile consisting of a series of Raman spectra recorded in the skin at a range of distances to the skin surface.

Raman measurements were conducted on the volar aspect of the forearm and on the thenar of the individuals. Laser light of 785 nm was focused in the skin and Raman spectra were recorded in the 400-1800 cm$^{-1}$ spectral region, herein defined as the "fingerprint region". The recorded depth range on the arm was 0 to 20 $\mu$m at 4 $\mu$m steps. The recorded depth ranges on the thenar were 30 to 50 $\mu$m at 10 $\mu$m steps. Measurements on the arm and on the thenar were repeated 10 times for each individual in between which the precise measurement location was changed slightly with translations in the order of 50-500 $\mu$m.

The wavenumber axis of the spectra was calibrated using the internal calibration standards of the Model 3510 Skin Composition Analyzer and instrument control software RiverICon (River Diagnostics). This software was also used to correct for the wavenumber dependent signal detection efficiency of the instrument.

From each measured Raman spectrum in the fingerprint region the NMF content was determined using the skin analysis software SkinTools 2.0 (River Diagnostics). The analysis method used by the software is described by Caspers et al. in J. Invest. Dermatol. 2001, 116, 434-442. The analysis method includes a classical least squares fitting step, in which a set of reference spectra of the major skin constituents is fitted to the Raman spectra of the arm or the palm, respectively. The fit coefficients are divided by the fit coefficient for the spectrum of keratin, which is the dominant dry mass fraction in the stratum corneum. This normalisation step corrects for variations in the absolute Raman intensity, which decreases with the distance of the laser focus position to the skin surface. The result of the analysis method is a measure of the relative NMF content, expressed in arbitrary units as an NMF to keratin ratio.

For each individual the average NMF content in the stratum corneum of the thenar was determined by calculating the numerical average of the relative NMF contents measured from 30-50 $\mu$m below the skin surface.

For each individual the average NMF content in the stratum corneum of the arm was determined from the relative NMF contents measured at 4 $\mu$m below the skin surface.

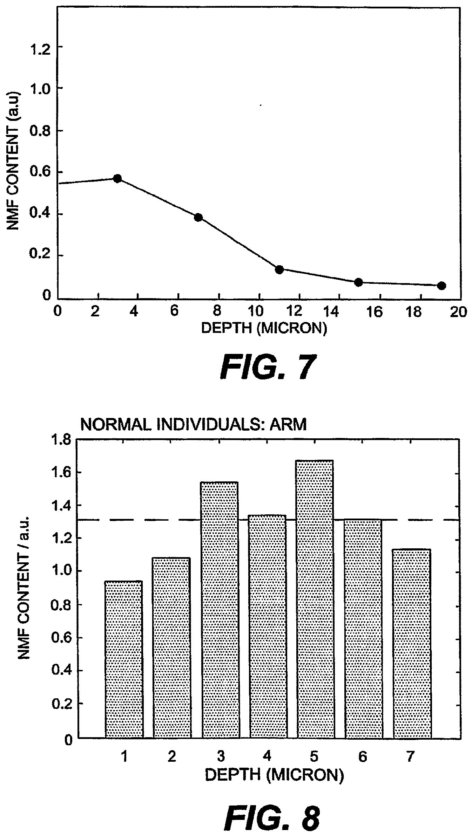

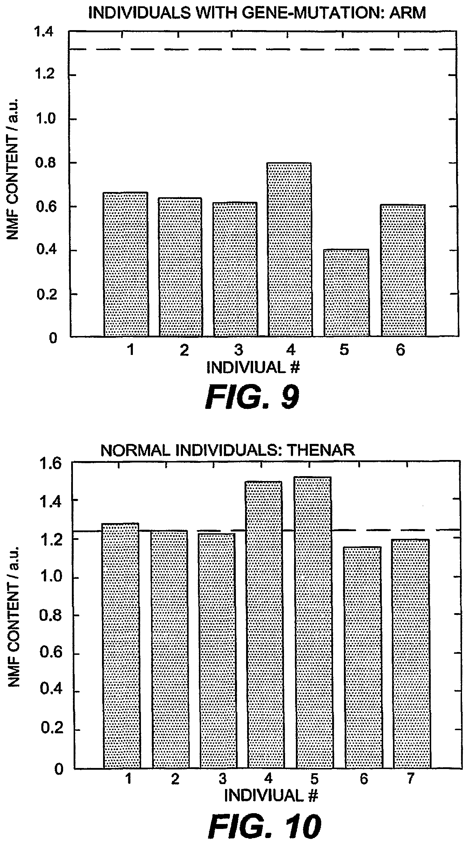

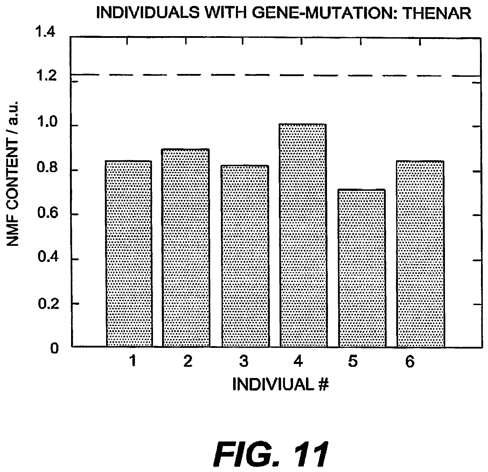

It was found that the NMF content found in the arms and in thenars of individuals with a filaggrin mutation is significantly lower than that in the arms and thenars of the control group without a filaggrin mutation. This is illustrated by Figures 6-11.

As shown by the example, the invention allows, despite the large biological variations in NMF content that have been reported, designing measurement protocols such that individuals with a potential loss-of-function mutation in the filaggrin gene, which predisposes for certain skin conditions, can be reliably identified.

**Claims**

1. A method for non-invasive determination of the potential presence of one or more loss-of-function mutation(s) in the gene encoding for filaggrin of an individual comprising

   (i) obtaining a vibrational spectrum from the stratum corneum of the individual;
   (ii) determining the local natural moisturising factor content from the obtained vibrational spectrum;
   (iii) optionally repeating steps (i) and (ii); and
   (iv) comparing the local natural moisturising factor content of the individual to a reference value,

   wherein the vibrational spectrum is obtained from stratum corneum of the thenar, 1-70 $\mu$m beneath the skin surface of the individual, preferably 2-50 $\mu$m,
   more preferably 3-30 $\mu$m or of the volar aspect of the forearm 1-10 $\mu$m beneath the skin surface of the individual, preferably 2-8 $\mu$m, more preferably 3-6 $\mu$m.

2. A method according to claim 1, wherein the vibrational spectrum is obtained *in vivo.*

3. A method according to claim 1, wherein the vibrational spectrum is obtained *ex vivo* on a stratum corneum sample that is taken from the individual.

4. A method according to any one of the preceding claims, wherein the vibrational spectrum is a Raman spectrum.

5. A method according to any one of the preceding claims, wherein the local natural moisturising factor content is determined from the intensity of the vibrational signal of NMF, relative to the intensity of the vibrational signal of an internal reference.

6. A method according to any one of the preceding claims, wherein steps i) and ii) as defined in claim 1 are repeated for at least 2 times, preferably at least 5 times, and more preferably 10 times.

7. A method according to any one of the preceding claims, wherein step (ii) as defined in claim 1 comprises fitting at least one reference spectrum of skin constituents to the obtained vibrational spectra.

8. A method according to claim 7, wherein the fit coefficients are divided by the fit coefficient of an internal reference spectrum.

9. A method according to claim 5 or 8, wherein the internal reference is keratin.

10. A method according to any one of the previous claims, for determining whether an individual is unsuitable, or at least less suitable, for a certain profession or activity.

11. A method according to any one of the previous claims, which method differentiates between a homozygous and a heterozygous loss-of-function mutation in the filaggrin gene of an individual, comprising classifying the loss-of-function mutation as a function of the natural moisturising factor content.


**Patentansprüche**

1. Verfahren zur nicht-invasiven Bestimmung des potentiellen Vorhandenseins einer oder mehrerer Funktionsverlustmutation(en) in dem Gen, kodierend für Filaggrin eines Individuums, umfassend

   (i) Erhalten eines Schwingungsspektrums aus dem Stratum corneum des Individuums;
   (ii) Bestimmen des lokalen natürlichen Feuchtigkeitsfaktor-Gehalts aus dem erhaltenen Schwingungsspektrum;
   (iii) optional Wiederholen der Schritte (i) und (ii); und
   (iv) Vergleichen des lokalen natürlichen Feuchtigkeitsfaktor-Gehalts des Individuums mit einem Referenzwert,

   wobei das Schwingungsspektrum aus Stratum corneum des Thenars, 1-70 $\mu$m unterhalb der Hautoberfläche des Individuums, vorzugsweise 2-50 $\mu$m, bevorzugter 3-30 $\mu$m oder des volaren Aspekts des Unterarms 1-10 $\mu$m unterhalb der Hautoberfläche des Individuums, vorzugsweise 2-8 $\mu$m, bevorzugter 3-6 $\mu$m, erhalten wird.

2. Verfahren nach Anspruch 1, wobei das Schwingungsspektrum in *vivo* erhalten wird.

3. Verfahren nach Anspruch 1, wobei das Schwingungsspektrum *ex vivo* auf einer Stratum corneum-Probe erhalten wird, die dem Individuum entnommen wird.

4. Verfahren nach einem der vorhergehenden Ansprüche, wobei das Schwingungsspektrum ein Raman-Spektrum ist.

5. Verfahren nach einem der vorhergehenden Ansprüche, wobei der lokale natürliche Feuchtigkeitsfaktor-Gehalt aus der Intensität des Schwingungssignals des NMF relativ zu der Intensität des Schwingungssignals einer internen Referenz bestimmt wird.

6. Verfahren nach einem der vorhergehenden Ansprüche, wobei die Schritte i) und ii) wie in Anspruch 1 definiert mindestens zweimal, vorzugsweise mindestens fünfmal und bevorzugter zehnmal wiederholt werden.

7. Verfahren nach einem der vorhergehenden Ansprüche, wobei Schritt (ii), wie in Anspruch 1 definiert, Anpassen von mindestens einem Referenzspektrum von Hautbestandteilen an die erhaltenen Schwingungsspektren umfasst.

8. Verfahren nach Anspruch 7, wobei die Anpassungskoeffizienten durch den Anpassungskoeffizienten eines internen Referenzspektrums geteilt werden.

9. Verfahren nach Anspruch 5 oder 8, wobei die interne Referenz Keratin ist.

10. Verfahren nach einem der vorhergehenden Ansprüche zum Bestimmen, ob ein Individuum für einen bestimmten Beruf oder Tätigkeit ungeeignet oder mindestens weniger geeignet ist.

11. Verfahren nach einem der vorhergehenden Ansprüche, welches Verfahren zwischen einer homozygoten und einer heterozygoten Funktionsverlustmutation im Filaggrin-Gen eines Individuums unterscheidet, umfassend Klassifizieren der Funktionsverlustmutation als Funktion des natürlichen Feuchtigkeitsfaktor-Gehalts.

**Revendications**

1. Procédé pour la détermination non invasive de la présence potentielle d'une ou plusieurs mutations avec perte de fonction dans le gène codant la filaggrine d'un individu comprenant

(i) l'obtention d'un spectre vibrationnel à partir de la couche cornée de l'individu ;
(ii) la détermination de la teneur en facteur naturel d'hydratation locale d'après le spectre vibrationnel obtenu ;
(iii) éventuellement la répétition des étapes (i) et (ii) ; et
(iv) la comparaison de la teneur en facteur naturel d'hydratation locale de l'individu à une valeur de référence,

où le spectre vibrationnel est obtenu à partir de la couche cornée de la paume, 1 - 70 $\mu$m en dessous de la surface de la peau de l'individu, de préférence 2 - 50 $\mu$m, de préférence encore 3 - 30 $\mu$m ou de la face antérieure de l'avant-bras 1 - 10 $\mu$m en dessous de la surface de la peau de l'individu, de préférence 2 - 8 $\mu$m, de préférence encore 3 - 6 $\mu$m.

2. Procédé selon la revendication 1, où le spectre vibrationnel est obtenu *in vivo.*

3. Procédé selon la revendication 1, où le spectre vibrationnel est obtenu *ex vivo* sur un échantillon de couche cornée qui est prélevé sur l'individu.

4. Procédé selon l'une quelconque des revendications précédentes, où le spectre vibrationnel est un spectre Raman.

5. Procédé selon l'une quelconque des revendications précédentes, où la teneur en facteur naturel d'hydratation locale est déterminée d'après l'intensité du signal vibrationnel du FNH, par rapport à l'intensité du spectre vibrationnel d'une référence interne.

6. Procédé selon l'une quelconque des revendications précédentes, où les étapes i) et ii) telles que définies dans la revendication 1 sont répétées au moins 2 fois, de préférence au moins 5 fois et de préférence encore 10 fois.

7. Procédé selon l'une quelconque des revendications précédentes, où l'étape (ii) telle que définie dans la revendication 1 comprend l'ajustement d'au moins un spectre de référence de constituants de la peau aux spectres vibrationnels obtenus.

8. Procédé selon la revendication 7, où les coefficients d'ajustement sont divisés par le coefficient d'ajustement d'un spectre de référence interne.

9. Procédé selon la revendication 5 ou 8, où la référence interne est la kératine.

10. Procédé selon l'une quelconque des revendications précédentes, pour déterminer si un individu n'est pas approprié, ou au moins moins approprié, pour une certaine profession ou activité.

11. Procédé selon l'une quelconque des revendications précédentes, lequel procédé différencie entre une mutation avec perte de fonction homozygote et une mutation avec perte de fonction hétérozygote dans le gène de la filaggrine d'un individu, comprenant la classification de la mutation avec perte de fonction en fonction de la teneur en facteur naturel d'hydratation.

**FIG. 1**

**FIG. 2**

EP 2 175 778 B1

**FIG. 3**

RAMAN SPECTRUM OF NMF

**FIG. 4**

13

RAMAN SPECTRUM OF SKIN

## FIG. 5

## FIG. 6

**FIG. 7**

**FIG. 8**

INDIVIDUALS WITH GENE-MUTATION: ARM

**FIG. 9**

NORMAL INDIVIDUALS: THENAR

**FIG. 10**

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030124553 A

### Non-patent literature cited in the description

- **ROLL et al.** *Curr. Opin. Allergy Clin. Immunol.,* 2004, vol. 4 (5), 373-378
- **STEMMLER et al.** *J. Invest. Dermatol.,* 2007, vol. 127, 722-724
- **ROLL et al.** *Curr, Opin. Allergy Clin. Immunol.,* 2004, vol. 4 (5), 373-378
- **SANDILANDS et al.** *J. Invest. Dermatol.,* 2006, vol. 126, 1770-1775
- **SANDILANDS et al.** *Nature Genetics,* 2006, vol. 38, 337-342
- **SMITH et al.** *Nature Genetics,* 2007, vol. 39, 650-654
- **IRVINE et al.** *J. Invest. Dermatol.,* 2006, vol. 126, 1200-1202
- **SCOTT et al.** *Biochim. Biophys. Acta,* 1982, vol. 719 (1), 110-117
- **RAWLINGS et al.** *J. Invest. Dermatol.,* 1994, vol. 103, 731-741
- **VAN DER POL et al.** *Abstract IFSCC meeting,* 24 September 2007
- **VAN DER POL et al.** *Abstract ISBS National meeting,* 12 October 2006
- **BOMMANNAN et al.** *J. Invest. Dermatol.,* 1990, vol. 95 (4), 403-408
- **CASPERS et al.** *J. Invest. Dermatol.,* 2001, vol. 116, 434-442
- **CASPERS et al.** *Biospectroscopy,* 1998, vol. 4, 31-39